# EUROPEAN PATENT APPLICATION

(11) **EP 3 804 529 A1**
(43) Date of publication of application: **14.04.2021**
(21) Application number: 19815697.8
(22) Date of filing: 15.07.2019
(51) Int. Cl.: A23K 20/111, A23K 10/20, A61K 31/11

(54) **APPLICATION OF CINNAMALDEHYDE AND/OR ALLANTOIN TO REPLACE HIGH ZINC AMOUNTS IN SUCKLING PIGLET FEED, FEED ADDITIVE OR ADDITIVE PREMIX, AND FEED**

(30) Priority: 08.06.2018 CN 201810589364
(71) Applicant: Hunan Jingtian Technology Industry Co., Ltd., Hunan 410000, (CN)
(72) Inventor: ZHOU, Jiancheng, Changsha, Hunan 410000 (CN)
(74) Representative: Eisenführ Speiser
(86) International application number: PCT/CN2019/095941
(87) International publication number: WO 2019/233496

(57) **Abstract**

Provided is an application of cinnamaldehyde and/or allantoin to partially replace or fully replace high zinc amounts in suckling piglet feed. Also provided are a feed additive or additive premix which uses cinnamaldehyde and/or allantoin to partially replace or fully replace high zinc amounts, and a suckling piglet feed which uses the feed additive or additive premix. The application of cinnamaldehyde and/or allantoin, the feed additive or additive premix and the feed reduce or remove the use of high zinc amounts in feed, and also have the effects of resisting diarrhea and promoting pig growth and development.

## Description

The application claims the priority to Chinese Patent Application No. 201810589364.6, titled "APPLICATION OF CINNAMALDEHYDE AND/OR ALLANTOIN TO REPLACE HIGH ZINC AMOUNTS IN SUCKLING PIGLET FEED, FEED ADDITIVE OR ADDITIVE PREMIX, AND FEED", filed on June 8, 2018 with the China National Intellectual Property Administration, which is incorporated herein by reference in entirety.

### FIELD

This application relates to the technical field of feed additives, and in particular relates to uses of cinnamylaldehyde and/or allantoin for replacing high amount of zinc in piglet feeds, feed additives or additive premixs, and feeds.

### BACKGROUND

In 1989, National Academy of Animal Sciences of Denmark found that adding 2,500-4,000 mg/kg of zinc oxide to the diet of early-weaned piglet could significantly reduce the diarrhea rate of piglets. Since then, a large number of studies and production practices have demonstrated that adding high amount of zinc can prevent piglet diarrhea effectively, with a low cost and good economy. Therefore, in order to ensure the safety of piglet weaning and prevent it from diarrhea in a large area, so as to cause a large area of dead pigs, the diet of weaned piglet has been supplemented with a high dose of zinc in most countries including China, so as to prevent piglets from diarrhea and death and increse profits from pig raising.

The high amount of zinc in feed mainly comes from zinc oxide or basic zinc chloride, which may cause contamination by heavy metal of zinc, including cadmium contamination from zinc ores, thus every country is making effort to reduce the amount of zinc added in feeds. The permitted maximum concentration of zinc in feed has been reduced from 3,000 ppm at initial to 2,250 ppm. In China, according to the new regulations to be implemented, the maximum concentration of zinc will be reduced further to 1600 ppm.

However, in practice, a stable anti-diarrhea effect can only be achieved when the weaned piglet feed contains a high doge 3,000-4,000 g/ton of zinc oxide (i.e., a zinc content of 2,250-3,000 ppm). If zinc is added at a maximum amount of 1600 ppm according to the new regulations, the diarrhea rate of piglets would reach 6-15%, which can not meet the requirements in market. Therefore, how to reduce the amount of zinc used in the feed and ensure the diarrhea rate of piglets within the control range is an urgent problem for those skilled in the art..

### SUMMARY

To solve the above technical prolems, the first object of the present invention is to provide use of cinnamylaldehyde and/or allantoin for replacing high amount of zinc in a piglet feed; the second object of the present invention is to provide a feed additive or additive premix in which high amount of zinc is partially or completely replaced by use of cinnamylaldehyde and/or allantoin; and the the third object of the present invention is to provide a piglet feed containg the feed additive or additive premix described above.

The present disclosure provides the following technical solutions.

Provided is use of cinnamylaldehyde and/or allantoin for partially or completely replacing high amount of zinc in a piglet feed.

Provided is use of stabilized cinnamylaldehyde and/or allantoin for partially or completely replacing high amount of zinc in a piglet feed.

Provided is a feed additive or additive premix for partially replacing high amount of zinc, based on the amount of complete formula feed, comprising 111-1600 ppm of zinc, and further comprising any one of a composition of 60-5000 ppm of cinnamylaldehyde and 10-5000 ppm of allantoin, 60-5000 ppm of cinnamylaldehyde, and 10-5000 ppm of allantoin.

Preferably, the above feed additive or additive premix, based on the amount of complete formula feed, comprises 111-1600 ppm of zinc, and further comprises any one of a composition of 60-600 ppm of cinnamylaldehyde and 10-400 ppm of allantoin, 60-600 ppm of cinnamylaldehyde, and 10-400 ppm of allantoin.

Provided is a feed additive or additive premix for completely replacing high amount of zinc, based on the amount of complete formula feed, comprising any one of a composition of 60-5000 ppm of cinnamylaldehyde and 10-5000 ppm of allantoin, 60-5000 ppm of cinnamylaldehyde, and 10-5000 ppm of allantoin.

Preferably, the above feed additive or additive premix, based on the amount of complete formula feed, comprises any one of a composition of 60-600 ppm of cinnamylaldehyde and 10-400 ppm of allantoin, 200-600 ppm of cinnamylaldehyde, and 50-400 ppm of allantoin.

Preferably, in the above feed additive or additive premix, the cinnamylaldehyde is stabilized cinnamylaldehyde; and the stabilized cinnamylaldehyde is specificailly any one or more selected from coated cinnamylaldehyde, chemically modified cinnamylaldehyde, and chemically modified and coated cinnamylaldehyde.

Preferably, the coated cinnamylaldehyde is specificailly obtained by coating the cinnamylaldehyde with a coating material; the chemically modified cinnamylaldehyde is specificailly produced by condensing cinnamylaldehyde with a compound containing an amino or imino group; and the chemically modified and coated cinnamylaldehyde is specifically obtained by condensing cinnamylaldehyde with a compound containing an amino or imino group and then coating with a coating material.

Preferably, the compound containing an amino or imino group is specificailly any one selected from oligochitosan, chitosan, lysine, methionine, threonine, tryptophan, glycine, aspartic acid, ethylenediamine, astragalus polysaccharide, cysteamine, glutamic acid, glutamine, antimicrobial peptides, aneurine hydrochloride, L-carnitine, DL-carnitine, niacinamide, ammonium bicarbonate, ammonium dihydrogen phosphate, diammonium hydrogen phosphate, isobutylidene diurea, urea phosphate, ammonium chloride, ammonium alginate, arginine, valine, leucine, isoleucine, methionine hydroxy analogue, tyrosine, taurine, N-carbamylglutamate, histidine, proline, phenylalanine, thiamine nitrate, cysteine, isobutylidene diurea, alginate oligosaccharide, nosiheptide, zinc bacitracin, amprolium hydrochloride ethoxy amide, robenidine hydrochloride, dinitolmide, bacitracin methylene disalicylate, xylooligosaccharide, chitosan oligosaccharide, galactomannan oligosaccharide, fructo-oligosaccharide, mannan oligosaccharide, alpha-cycloalanine, N,O-carboxymethyl oligochitosan, oligo β-(1,4)-2-amino-2-deoxy-glucose, guanidinoacetic acid, 8-methyl-N-vanillyl-L-nonenamide, capsaicine, dihydrocapsaicin, nordihydrocapsaicin, homocapsaicin, homodihydrocapsaicin I, homodihydrocapsaicin II, nonoyl vanillylamide, decoyl vanillylamide, and capryl vanillylamide.

Preferably, the coating material is one or more selected from cyclodextrin, polyethylene glycol, polyvinyl alcohol, polyacrylic resins, hydroxypropyl methylcellulose phthalate, hydroxypropyl methylcellulose acetate, ethyl succinate butyrate, tributyrin, chitosan, pullulan, shellac, polyvinyl alcohol acetate phthalate, cellulose and derivatives thereof, cellulose acetate phthalate, cellulose acetate 1,2,4-benzenetricarboxylate, hydroxypropyl methylcellulose 1,2,4-benzenetricarboxylate, cellulose acetate succinate, hydroxypropyl cellulose acetate succinate, methacrylate, methacrylic acid, acrylate, butyl acrylate, acrylic resins, ethyl cellulose, glycerol triacetate, vinyl propionate, poly(vinyl propionate), poly(vinyl acetate), poly(vinyl butyrate), polymers of vinyl carboxylate, vinyl acrylate, polymers of vinylpyrrolidone and vinyl carboxylate, polymers of vinyl acrylate, vinyl methacrylate and polymers thereof and ethyl o-benzoate.

Provided is a piglet feed, containing any one of the feed additive or additive premix described above.

In view of the problems in prior art, the present disclouse provides use of cinnamylaldehyde and/or allantoin for replacing high amount of zinc in a feed.

In the early years, cinnamylaldehyde was proposed for preventing moulds in feed, but because the cinnamylaldehyde is very unstable, no one actually applies it to feed production to prevent moulds in feed, and reports are limited to test results. In recent years, due to the increasing demand for the prohibition of antibiotics, and the stabilization technology of cinnamaldehyde has been improved to a certain extent. At present, it is more common in the market that cyclodextrin-coated stabilized cinnamaldehyde replaces antibiotics. The addition amount of cinnamaldehyde is usually low (less than 60ppm), and the cinnamaldehyde and high amount of zinc in the feed are added at the same time, so that the feed has a more stable anti-diarrhea effect. However, so far, no one has proposed to use cinnamylaldehyde to replace the high amount of zinc. Similarly, no one has proposed to use allantoin or a composition of cinnamylaldehyde and allantoin to replace the high amount of zinc. In the present disclosure, cinnamylaldehyde and/or allantoin is used to partially or completely replace the high amount of zinc, so that the feed with sufficient amount of cinnamylaldehyde and/or allantoin added, without need of adding high amount of zinc, only with the addition of a necessary amount of zinc (60-110 ppm) required for animal nutrition, can produce an anti-diarrhea effect.

Cinnamylaldehyde can promote the release of growth factors for vascular endothelial cells and epithelial cells of the digestive tract mucosa, timely repair the undeveloped digestive tract mucosa damaged by allergenic substances in feed, especially the small intestinal chorion. It can inhibit inflammation and its over expression, and at the same time directly kill most of fungi, including aspergillus flavus, and protozoa as well as viruses and bacteria, and prevent diarrhea caused by secondary infection after the damage of the digestive tract mucosa. Therefore, cinnamylaldehyde can reduce the incidence of the injury at the digestive tract mucosa, particularly chorion of small intestine, reduce diarrhea greatly, and prevent secondary infection, thereby achieving a good anti-diarrhea effect, and help piglets grow quickly. The piglets fed with cinnamaldehyde instead of high amount of zinc feed can gain 12-30% faster in weight than piglets using diets of high amount of zinc in the late nursery and little piglet feed stage, which opened a new era in the development of the pig industry and feed industry.

Allantoin has the same function of repairing damaged mucosa and chorion as the cinnamylaldehyde, but it also has a certain astringent effect. Therefore, allantoin also plays a role in replacing high amount of zinc. In addition, in a composition of allantoin and cinnamylaldehyde, the cinnamylaldehyde can have a better reinforcing and repairing effect when low temperature and sudden temperature drop.

When the cinnamaldehyde exceeds a certain dose in the feed, the growth of osteoblasts and fibroblasts will be blocked, which will affect the growth and development of pigs (but still better than the high-zinc group, high amount of zinc will also affect the growth and development of pigs in the later period), and the use of allantoin alone or in large doses will not achieve the growth-promoting and antibacterial effects of cinnamaldehyde at an appropriate dose. Therefore, using suitable stabilized cinnamaldehyde alone in seasons with good heat preservation and higher temperatures can partially or completely replace high amount of zinc, and can ensure safety, and there will be no diarrhea and death of piglets that are unsatisfactory to farmers. In the case of poor heat preservation or sudden temperature drop and lack of emergency plans, using a composition of cinnamaldehyde and allantoin that has a sufficient dose but does not affect (late) growth is a better solution. It can also partially or completely replace high amount of zinc to obtain very low piglet diarrhea rate and almost no deaths that are satisfactory to farmers, and it will not bring adverse effects on the later development of piglets.

### BRIEF DESCRIPTION OF DRAWINGS

In order to more clearly describe the technical solutions in the embodiments of this application or the prior art, hereinafter the drawings that need to be used to describe the embodiments or the prior art will be briefly introduced. Obviously, the drawings described below are only part of the embodiments in this application. For a person having ordinary skill in the art, other drawings can be obtained based on these drawings without creative work.
Figure 1 is a photo of 0% diarrhea of suckling piglets in Example 1 of the present invention.
Figure 2 is a photo of 100% diarrhea of suckling piglets in Example 1 of the present invention.

### DETAILED DESCRIPTION

In order to enable those skilled in the art to better understand the technical solutions in this application, hereinafter the technical solutions in the embodiments of this application will be described clearly and completely, in conjunction with the drawings in the embodiments of this application. Obviously, the described embodiments are only part of the embodiments of this application, rather than all the embodiments. Based on the embodiments in this application, all other embodiments obtained by a person having ordinary skill in the art without creative work should fall within the protection scope of this application.

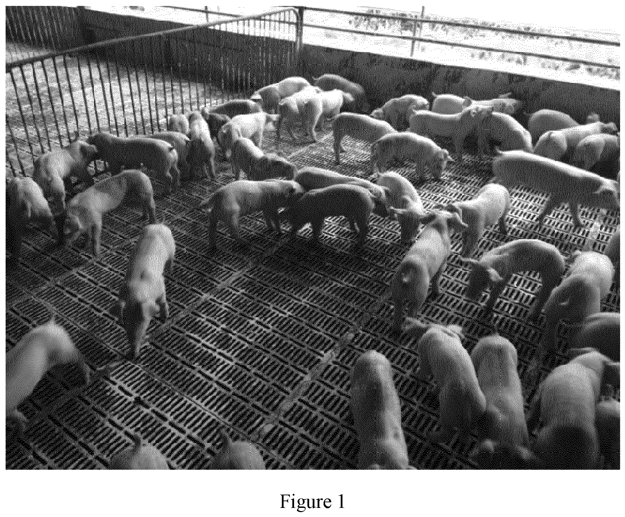

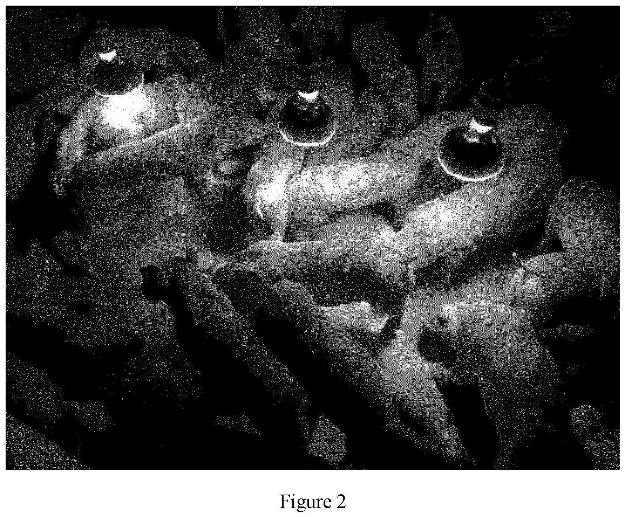

As shown in Figure 1 and Figure 2, the present disclosure provides the following technical solutions.

Provided is use of cinnamylaldehyde and/or allantoin for partially or completely replacing high amount of zinc in a piglet feed.

When a piglet is weaned, it transfers from eating breast milk to eating artificial feed. There are many anti-nutritional factors in the feed, such as proteinase inhibitors, soybean allergic proteins, soybean trypsin inhibitors, exogenous corrosive phenols and acids, high-dose metal additives, hard crude fibers, lignin, and insoluble mineral raw materials, as well as bacteria, fungi, molds, viruses, protozoa ingested with feed, will damage the developing digestive tract mucosa in piglets, particularly the underdeveloped small intestinal chorion, and further cause inflammation, diarrhea in weaned piglets, and secondary diseases. This is an important factor that makes it difficult for piglets to get through the weaning period. A piglet can better resist these negative factors only after 65-70 days old when the intestine is well developed.

In the prior art, a sufficient dose of high amount of zinc has been used as feed additives, to achieve an anti-diarrhea effect. For example, 21-33 days old weaned pigs are feed with creep feed with addition of 2,250-3,000 ppm of zinc (zinc oxide) and nursery feed with addition of 1600-1,875 ppm of zinc (zinc oxide). A sufficient dose of high amount of zinc has sufficient astringent effects, which can make the digestive tract mucosal cells in the piglet, especially the small intestine chorionic cells, converge and shrink, thereby avoiding the damage caused by the diarrhea-causing substances in feed, preventing piglet from diarrhea, and greatly reducing the incidence of secondary infections, diseases and death. However, it is precisely because of such astringent effects of zinc oxide, a long term use will hinder the growth and development of the digestive tract mucosa, especially the small intestinal chorion, and cause growth retardation. Such hindering effects of zinc oxide is particulary obvious in the late nursery and feed stage of little piglets.

However, the high amount of zinc in feed mainly comes from zinc oxide or basic zinc chloride, which may cause heavy metal pollution to the environment, and a high amount of zinc is also accompanied by cadmium pollution, causing seriously impact on the environment. Currently, every country is making effort to reduce the amount of zinc added in feeds, but because adding high zinc to feed is a stable and very effective solution for suckling piglets to wean and resist diarrhea, it is difficult to find a substitute. Although there have been reports of replacing high zinc with nano-zinc oxide, coated zinc oxide, and basic zinc chloride. Even if they have effects, the effects are based on a certain amount of zinc oxide, such as using zinc oxide with a zinc concentration of 300-600ppm. Moreover, they still cannot protect the piglets from diarrhea and death at low temperature or sudden cooling like high amount of zinc. Conversely, they may result in diarrhea and death in most of piglets. It is a worldwide problem to not use high amount of zinc in diets for weaned piglets. Even in the European Union, which has the most developed pig farming industry, it is only planed to ban the use of high amount of zinc in the next five years. In China, the allowable zinc content in feed is also gradually reducing.

In view of the problems in the prior art, the present disclouse provides use of cinnamylaldehyde and/or allantoin for replacing high amount of zinc in a feed.

In the early years, cinnamylaldehyde was proposed for preventing moulds in feed, but because the cinnamylaldehyde is very unstable, no one actually applies it to feed production to prevent moulds in feed, and reports are limited to test results. In recent years, due to the increasing demand for the prohibition of antibiotics, and the stabilization technology of cinnamaldehyde has been improved to a certain extent. At present, it is more common in the market that cyclodextrin-coated stabilized cinnamaldehyde replaces antibiotics. The addition amount of cinnamaldehyde is usually low (less than 60ppm), and the cinnamaldehyde and high amount of zinc in the feed are added at the same time, so that the feed has a more stable anti-diarrhea effect. However, so far, no one has proposed to use cinnamylaldehyde to replace the high amount of zinc. Similarly, no one has proposed to use allantoin or a composition of cinnamylaldehyde and allantoin to replace the high amount of zinc. In the present disclosure, cinnamylaldehyde and/or allantoin is used to partially or completely replace the high amount of zinc, so that the feed with sufficient amount of cinnamylaldehyde and/or allantoin added, without need of adding high amount of zinc, only with the addition of a necessary amount of zinc (60-110 ppm) required for animal nutrition, can produce an anti-diarrhea effect.

Cinnamylaldehyde can promote the release of growth factors for vascular endothelial cells and epithelial cells of the digestive tract mucosa, timely repair the undeveloped digestive tract mucosa damaged by allergenic substances in feed, especially the small intestinal chorion. It can inhibit inflammation and its over expression, and at the same time directly kill most of fungi, including aspergillus flavus, and protozoa as well as viruses and bacteria, and prevent diarrhea caused by secondary infection after the damage of the digestive tract mucosa. Therefore, cinnamylaldehyde can reduce the incidence of the injury at the digestive tract mucosa, particularly chorion of small intestine, reduce diarrhea greatly, and prevent secondary infection, thereby achieving a good anti-diarrhea effect, and help piglets grow quickly. The piglets fed with cinnamaldehyde instead of high amount of zinc feed can gain 12-30% faster in weight than piglets using diets of high amount of zinc in the late nursery and little piglet feed stage, which opened a new era in the development of the pig industry and feed industry.

Allantoin has the same function of repairing damaged mucosa and chorion as the cinnamylaldehyde, but it also has a certain astringent effect. Therefore, allantoin also plays a role in replacing high amount of zinc. In addition, in a composition of allantoin and cinnamylaldehyde, the cinnamylaldehyde can have a better reinforcing and repairing effect when low temperature and sudden temperature drop.

When the cinnamaldehyde exceeds a certain dose in the feed, the growth of osteoblasts and fibroblasts will be blocked, which will affect the growth and development of pigs (but still better than the high-zinc group, high amount of zinc will also affect the growth and development of pigs in the later period), and the use of allantoin alone or in large doses will not achieve the growth-promoting and antibacterial effects of cinnamaldehyde at an appropriate dose. Therefore, using suitable stabilized cinnamaldehyde alone in seasons with good heat preservation and higher temperatures can partially or completely replace high amount of zinc, and can ensure safety, and there will be no diarrhea and death of piglets that are unsatisfactory to farmers. In the case of poor heat preservation or sudden temperature drop and lack of emergency plans, using a composition of cinnamaldehyde and allantoin that has a sufficient dose but does not affect (late) growth is a better solution. It can also partially or completely replace high amount of zinc to obtain very low piglet diarrhea rate and almost no deaths that are satisfactory to farmers, and it will not bring adverse effects on the later development of piglets.

Provided is use of stabilized cinnamylaldehyde and/or allantoin for partially or completely replacing high amount of zinc in a piglet feed.

Preferably, stabilized cinnamylaldehyde is used as an alternative product for high amount of zinc. The stabilized cinnamylaldehyde may be used alone or in combination with allantoin. Allantoin also can be used alone. The stabilized cinnamylaldehyde is specificailly any one or more selected from coated cinnamylaldehyde, chemically modified cinnamylaldehyde, and chemically modified and coated cinnamylaldehyde. The cinnamylaldehyde after stabilization treatment can overcome the instability of the cinnamylaldehyde. In the process of feed preparation, especially pelleting, the content of cinnamaldehyde can be kept stable. After the preparation of feed and long-term storage, it can still contain enough cinnamaldehyde.

Provided is a feed additive or additive premix for partially replacing high amount of zinc, which based on the amount of complete formula feed, comprises 111-1600 ppm of zinc, and further comprises any one of a composition of 60-5000 ppm of cinnamylaldehyde and 10-5000 ppm of allantoin, 60-5000 ppm of cinnamylaldehyde, and 10-5000 ppm of allantoin.

Preferably, the feed additive or additive premix, based on the amount of complete formula feed, comprises 111-1600 ppm zinc, and further comprises any one of a composition of 60-600 ppm of cinnamylaldehyde and 10-400 ppm of allantoin, 60-600 ppm of cinnamylaldehyde, and 10-400 ppm allantoin.

The present disclosure further provides a feed additive or additive premix for partially replacing high amount of zinc. Partially replacing high amount of zinc means the feed additive or additive premix still contains 111-1600 ppm of zinc, based on the amount of complete formula feed. Less than 110 ppm, such as 60-110 ppm, is the range where zinc needs to be added as a nutrient in the feed, and does not belong to the range of high zinc amount added for diarrhea. The currently used high zinc amount is usually within 1600-3000ppm, calculated as (oxide) zinc, in order to play the role of anti-diarrhea. When the feed additive provided by the present invention is used to partially replace high amount of zinc, the feed additive contains 111-1600 ppm of zinc, and further comprises includes any one of cinnamaldehyde, allantoin, and a combination of cinnamaldehyde and allantoin.

When cinnamylaldehyde is used alone, based on the amount of formula feed, the feed additive or additive premix comprises 111-1600 ppm of zinc, and further comprises 60-5000 ppm of cinnamylaldehyde, preferably 60-600 ppm, more preferably 60-300 ppm, and even more preferably 100-200 ppm of cinnamylaldehyde. When allantoin is used alone, based on the amount of complete formula feed, the feed additive or additive premix comprises 111-1600 ppm of zinc, and further comprises 10-5000 ppm of allantoin, preferably 10-400 ppm, more preferably 50-300 ppm, and even more preferably 100-200 ppm of allantoin. When the composition of cinnamylaldehyde and allantoin is used, based on the amount of complete formula feed, the feed additive or additive premix comprises 111-1600 ppm of zinc, and further comprises a composition of 60-5000 ppm of cinnamylaldehyde and 10-5000 ppm of allantoin, preferably a composition of 60-600 ppm of cinnamylaldehyde and 10-400 ppm of allantoin, more preferably a composition of 60-300 ppm of cinnamylaldehyde and 50-300 ppm of allantoin, and even more preferably a composition of 100-200 ppm of cinnamylaldehyde and 100-200 ppm of allantoin.

Provided is a feed additive or additive premix for completely replacing high amount of zinc, which based on the amount of complete formula feed, comprises any one selected from a composition of 60-5000 ppm of cinnamylaldehyde and 10-5000 ppm of allantoin, 60-5000 ppm of cinnamylaldehyde, and 10-5000 ppm of allantoin.

Preferably, the feed additive or additive premix, based on the amount of complete formula feed, comprises any one of a composition of 60-600 ppm of cinnamylaldehyde and 10-400 ppm of allantoin, 200-600 ppm of cinnamylaldehyde, and 50-400 ppm of allantoin.

The present disclosure further provides a feed additive or additive premix for compeletly replacing high amount of zinc. Compeletly replacing high amount of zinc means that the feed additive contains no zinc at a dose for anti-diarrhea, and the feed still contains less than 110 ppm of zinc, such as 60-110 ppm, which is the range where zinc needs to be added as a nutrient in the feed, and does not belong to the range of high zinc amount added for diarrhea. When the feed additive provided by the present disclosure is used to compeletly replace the high amount of zinc, the feed additive contains any one of cinnamylaldehyde, allantoin and a composition of cinnamylaldehyde and allantoin.

When cinnamylaldehyde is used alone, based on the amount of complete formula feed, the feed additive or additive premix comprises 60-5000 ppm of cinnamylaldehyde, preferably 200-600 ppm, and more preferably 400-500 ppm of cinnamylaldehyde. When allantoinis is used alone, based on the amount of formula feed, the feed additive or additive premix comprises 10-5000 ppm of allantoin, preferably 10-400 ppm, and more preferably 50-300 ppm of allantoin. When a composition of cinnamylaldehyde and allantoin is used, based on the amount of formula feed, the feed additive or additive premix comprises a composition of 60-5000 ppm of cinnamylaldehyde and 10-5000 ppm of allantoin, preferably a composition of 60-600 ppm of cinnamylaldehyde and 10-400 ppm of allantoin, and more preferably a composition of 200-300 ppm of cinnamylaldehyde and 50-300 ppm of allantoin.

Preferably, in the feed additive or additive premix, the cinnamylaldehyde is stabilized cinnamylaldehyde; the stabilized cinnamylaldehyde is specificailly any one or more selected from coated cinnamylaldehyde, chemically modified cinnamylaldehyde, and chemically modified and coated cinnamylaldehyde.

Preferably, the coated cinnamylaldehyde is specificailly obtained by coating the cinnamylaldehyde with a coating material; the chemically modified cinnamylaldehyde is specificailly produced by condensing cinnamylaldehyde with a compound containing an amino or imino group; and the chemically modified and coated cinnamylaldehyde is specificailly obtained by condensing cinnamylaldehyde with a compound containing an amino or imino group and then coating with a coating material

Preferably, the compound containing an amino or imino group is specificailly any one selected from oligochitosan, chitosan, lysine, methionine, threonine, tryptophan, glycine, aspartic acid, ethylenediamine, astragalus polysaccharide, cysteamine, glutamic acid, glutamine, antimicrobial peptides, aneurine hydrochloride, L-carnitine, DL-carnitine, niacinamide, ammonium bicarbonate, ammonium dihydrogen phosphate, diammonium hydrogen phosphate, isobutylidene diurea, urea phosphate, ammonium chloride, ammonium alginate, arginine, valine, leucine, isoleucine, methionine hydroxy analogue, tyrosine, taurine, N-carbamylglutamate, histidine, proline, phenylalanine, thiamine nitrate, cysteine, isobutylidene diurea, alginate oligosaccharide, nosiheptide, zinc bacitracin, amprolium hydrochloride ethoxy amide, robenidine hydrochloride, dinitolmide, bacitracin methylene disalicylate, xylooligosaccharide, chitosan oligosaccharide, galactomannan oligosaccharide, fructo-oligosaccharide, mannan oligosaccharide, alpha-cycloalanine, N,O-carboxymethyl oligochitosan, oligo β-(1,4)-2-amino-2-deoxy-glucose, guanidinoacetic acid, 8-methyl-N-vanillyl-L-nonenamide, capsaicine, dihydrocapsaicin, nordihydrocapsaicin, homocapsaicin, homodihydrocapsaicin I, homodihydrocapsaicin II, nonoyl vanillylamide, decoyl vanillylamide, and capryl vanillylamide.

Preferably, the coating material is one or more selected from cyclodextrin, polyethylene glycol, polyvinyl alcohol, polyacrylic resins, hydroxypropyl methylcellulose phthalate, hydroxypropyl methylcellulose acetate, ethyl succinate butyrate, tributyrin, chitosan, pullulan, shellac, polyvinyl alcohol acetate phthalate, cellulose and derivatives thereof, cellulose acetate phthalate, cellulose acetate 1,2,4-benzenetricarboxylate, hydroxypropyl methylcellulose 1,2,4-benzenetricarboxylate, cellulose acetate succinate, hydroxypropyl cellulose acetate succinate, methacrylate, methacrylic acid, acrylate, butyl acrylate, acrylic resins, ethyl cellulose, glycerol triacetate, vinyl propionate, poly(vinyl propionate), poly(vinyl acetate), poly(vinyl butyrate), polymers of vinyl carboxylate polymer, vinyl acrylate, polymers of vinylpyrrolidone and vinyl carboxylate, polymers of vinyl acrylate, vinyl methacrylate and polymers thereof, and ethyl o-benzoate.

Preferably, in the feed additive provided by the present disclosure, the used cinnamylaldehyde is stabilized cinnamylaldehyde. The stabilized cinnamylaldehyde is specificailly any one or more selected from coated cinnamylaldehyde, chemically modified cinnamylaldehyde, and chemically modified and coated cinnamylaldehyde. Among them, the chemically modified cinnamylaldehyde is preferably produced by condensing cinnamylaldehyde with a compound containing an amino or imino group. The cinnamylaldehyde produced by condensation can be hydrolyzed to cinnamylaldehyde and the original amino-containing substances in an acidic environment, (e.g., in gastric juice environment), and is a cinnamylaldehyde in stabilized and slow-release form. The coated cinnamylaldehyde is the cinnamylaldehyde coated with a coating material. In addition, the cinnamylaldehyde obtained by chemical modification may further be coated to further improve the stability of cinnamylaldehyde.

Cinnamylaldehyde is unstable, and the feed containing unstabilized cinnamylaldehyde has a short storage time, thus it is usually prepared shortly before use in research experiments. The stabilized cinnamylaldehyde obtained by condensing with the substance containing an amino or imino group can keep stable in the feed preparation process and feed storage process, and the content of cinnamaldehyde is less reduced. Therefore, use of stabilized cinnamylaldehyde can produce an effect of stably replacing the high amount of zinc, which is beneficial to the industrial production of feed. Among the stabilized cinnamylaldehyde provided by the present disclosure, the cinnamylaldehyde produced by condensing cinnamylaldehyde with a substance containing an amino or imino group or the cinnamylaldehyde obtained by condensing cinnamylaldehyde with a substance containing an amino or imino group and then coating is preferably used, which remains stable in the feed preparation process. The cinnamylaldehyde obtained by condensing cinnamylaldehyde with a substance containing an amino or imino group used in the present disclosure remains 78% after drying in an oven at 130°C for 10 minutes, which can meet the requirement for most feed production processes, including the high-temperature production of aquatic feed. The nursery feed added with the stabilized cinnamylaldehyde at 400 g/ton lost only 1 g on the 30^{th} day, and only 9.05% on the 60^{th} day, which can meet the stability requirements of the additive in feed.

Provided is a piglet feed, containing any one of the feed additive or additive premix decribed above.

The present disclosure further provides a piglet feed used the above feed additive or additive premix. The feed itself contains 60-110 ppm of zinc as nutrients and others nutrients required for pig growth and development. The feed additive for partially or completely replacing high amount of zinc provided by the present disclosure is also added, which can ensure the suckling piglets to grow normally and quickly and at the same time produce an anti-diarrhea effect.

In the following creep feed test, the basal diet formulas, and the amount of trace elements and vitamin are as follows:

**Table 1 Basal diet formula (%)**

| | |
|---|---|
| Extruded corn | 22 |
| Extruded rice | 22 |
| 46% Soybean meal | 10 |
| Fish meal | 3 |
| Extruded soybean | 6 |
| Low protein whey powder | 10 |
| Plasma protein (Hubei NP) | 3 |
| Flour | 10 |
| Emulsified fat powder | 2 |
| Intestinal membrane protein (United States) | 3 |
| Glucose | 3 |
| Premix | 6 |
| Total | 100 |

**Table 2 Guaranteed values of trace elements in diet (mg/kg)**

| | |
|---|---|
| Copper | 10-30 |
| Iron | 80-120 |
| Zinc | 60-110 |
| Manganese | 20-30 |
| Iodine | 0.5-1.2 |
| Selenium | 0.3-0.5 |

**Table 3 Guaranteed values of vitamin in diet (Minimum amount per kilogram)**

| | | |
|---|---|---|
| Vitamin A | 9,750,000 | IU |
| Vitamin D3 | 3,000,000 | IU |
| Vitamin E | 24,000 | IU |
| Vitamin K3 | 3,000 | mg |
| Vitamin B1 | 3,000 | mg |
| Vitamin B2 | 7,500 | mg |
| Vitamin B6 | 2,400 | mg |
| Vitamin B12 | 22.5 | mg |
| D-pantothenic acid | 15,000 | mg |
| D-biotin | 180 | mg |
| Folic acid | 1,500 | mg |
| Niacin | 30,000 | mg |

### Example 1

In the pig farm of Guangmin breeding Co., Ltd. in Jinshi City, Changde, Hunan, 100 21 days old weaned piglets with the same parity sows and a similar weight of approximately 6.5kg were selected and randomly divided into two groups, with 50 piglets in each group, half male and half female. In the test group, the piglets were pre-fed with the creep feed produced according to the above formulas from 7 days old to weaning.

In the test group, the piglets were fed with a creep feed free of high amount of zinc (containing 60 ppm of Zn) and antibiotics, in which oligochitosan aminocinnamylaldehyde was added (the cinnamaldehyde content was 50 ppm, the 50 ppm of cinnamaldehyde in the feed had no loss during the 60-day storage period tested by a liquid chromatography). In the control group, the piglets were fed with a creep feed containing high amount of zinc (2,250 ppm) and antibiotics, produced by a company in Fujian province, and the pig house was warmed to above 22°C by floor heating. The results are shown below.

During the pre-feeding period and to the 7^{th} day after weaning, no piglet had diarrhea in the test group (see Figure 1), while 5 piglets had diarrhea in the control group. In the afternoon of the 8^{th} day, a sudden temperature drop caused the temperature in pig house at night to drop from 22°C to 16-18°C. At that night, the piglets in the test group began to have 100% diarrhea (see Figure 2), while no piglet had diarrhea in the control group. After treatment next day, the diarrhea in the test group dropped to about 10%, with 2 piglets dead. On the 3^{rd} day from that night, a sudden temperature drop caused the piglets in the test group to have 100% diarrhea again, with 4 piglets dead, and 6 piglets died on the 5^{th} day. However, no one piglet had diarrhea in the control group. In just five days, 12 piglets died in total, with a mortality rate of 24%. In the control group, neither diarrhea nor death occurred. On the 6^{th} day, the test had to be terminated. The piglets in the test group were fed with the feed used in the control group, and then the diarrhea was improved immediately, and no piglet died.

Conclusion: For weaned piglets of age, under the condition of constant heat preservation to meet the suitable growth temperature for piglets, the probability of piglets diarrhea is inherently low, thus both in the test group using 50 ppm of cinnamylaldehyde and the control group using 2,250 ppm of high amount of zinc, no diarrhea occured. However, when the temperature suddenly dropped, 100% diarrhea occurred in the test groups, indicating that 50 ppm of cinnamylaldehyde cannot replace the high amount of zinc.

As can be seen from Example 1, weaned piglets weighing lower than 30kg were only fed with 60-110 ppm of zinc according to the nutritional requirements of the piglets. The piglets will suffer from diarrhea at a rate up to 30-100% with a mortality rate up to 10-30% or even higher, once the temperature is lower than 20°C or drops suddenly, which seriously affects the efficiency of pig farming. In China, small and medium-sized pig farming factories and scattering raising-households account for the vast majority, and it is obviously difficult to keep the pig houses at a temperature of 20°C or higher. In the pig industry, air conditioners for constant heat preservation are not available, and the use of air conditioners may also encounter power outages or equipment failure, thus it is hard for an enterprise to avoid the sudden drop in temperature of pig houses. When the temperature in a pig house is lower than 20°C, not adding a high amount of zinc will definitely cause large-scale diarrhea and death of piglets. Thus, a high amout of zinc in the diet of the weaning piglets is regarded as "Patron saint" of preventing pigs from diarrhea.

### Example 2

In the pig farm of Changde Huisheng Group in Jiubu port, Taoyuan county, Changde city, Hunan province, in the test group, 200 21-28 days old weaned piglets were fed with a creep feed free of high amount of zinc (containing 60 ppm of Zn) at a temperature from -3°C to 14°C, in which oligochitosan aminocinnamylaldehyde was added (the cinnamaldehyde content was 50 ppm, the 50 ppm of cinnamaldehyde in the feed had no loss during the 60-day storage period tested by a liquid chromatography). As a result, 67 piglets died with a mortality rate of 33.5%. In the control group, 1700 21-28 days old weaned piglets were fed with a creep feed with high amount of zinc (containing 2,250 ppm of zinc) at a temperature from -3°C to 14°C. As a result, 275 piglets died and were eliminated with a mortality rate of 16.2%. The results in the control group were better than that in the test group, indicating that at a low temperature less than 50 ppm of cinnamylaldehyde in diet cannot replace high amount of zinc. The creep feed in basal diet for the test group and the control group is shown inTable 1.

### Example 3

In the pig farm of Changde Huisheng Group in Jiubu Port, Taoyuan County, Changde City, Hunan province, the production test on an antibiotic free low-zinc creep feed (containing 60 ppm of zinc) was conducted from January 12, 2018 to January 23, 2018.

A total of 420 21-25 days old of weaned piglets were kept at 23-25°C in the farrowing house where the sows were driven three days before, and then on the fourth day transferred to the nursery houses. All pigs in the nursery house were equipped with electric heating plates at 15 piglets/m², and the electric heating plate was kept at a temperature of 28-32°C. The piglets were divided into a test group and a control group. The test group had 370 piglets, which was fed with an antibiotic free low-zinc creep feed (containing 60 ppm of zinc) in which lysine coated aminocinnamylaldehyde (containing 60g/ton of cinnamylaldehyde; it was measured that the feed retained 59 g/ton of cinnamylaldehyde after 60 days) was added, and nutrient ingredients were given in Table 1, Table 1 and Table 3. The control group had 50 piglets, and the basal diet formula was given in Table 1, in which 2,250 ppm of high amount of zinc was added but no cinnamylaldehyde.

Experimental results: 420 piglets did not have diarrhea in the first four days. On the first, second, and third days of changing pigsty, they were fed with 50% of their feed intake, and the diarrhea rate in both groups was 0.5%. Due to the low temperature at the nursery houses, only 15-19°C (the suitable growth temperature for piglets should be 23-26°C), diarrhea began to increase after the fourth day of changing pigsty, but the test group had less diarrhea than the control group. On the eighth day, the diarrhea piglets were treated with 10% florfenicol injection at 3 ml/piglet, once a day, and the test ended after twelve days. As a result shown in Table 4, the daily weight gain in the test group was 16.73% higher than that in the control group.

**Table 4 A production test on the antibiotic free low-zinc creep feed (containing 60 ppm of zinc) in a pig farm in Jiubu Port, Taoyuan County, Changde City**

| | Jinglvan cinnamylaldehyde creep feed (370 piglets) | High-zinc antibiotic control group (50 piglets) | Temperature of the piglet house (°C) |
|---|---|---|---|
| Additives g/ton | Zinc 60, Cinnamylaldehyde 60 | Zinc 2,250, Cinnamylaldehyde 0 | |
| Initial weight after weaning in kg | 7.32 | 7.49 | |
| Incidence of diarrhea (1.12- 1.15) | 0 | 0 | 23-25 |
| Incidence of diarrhea (1.16- 1.17) | 0.5% | 0.5% | 17-25 |
| Incidence of diarrhea (1.18- 1.23) | 5.60% | 6.67% | 15-19 |
| Final weight in kg | 11.13 | 10.76 | |
| Daily weight gain in g | 317.5 | 272 | |
| Increment in daily weight gain | 16.73% | | |

Example 3 and Table 4 show that 60 mg/kg of cinnamylaldehyde can be used to compeletly replace the high amount of zinc in feed, when the temperature is above 15°C and the abdomen of the pig is kept at 28-32°C using a heating plate.

### Example 4

240 21 days old weaned primiparous three-way crossbred piglets were divided into six groups, in which those having diarrhea (diseases) were not treated. The temperature of the pig houses were 24°C. Except for zinc oxide, the basal diet, vitamin and trace elements for all of the six groups were given as shown in Table 1, 2 and 3, and no acidulant was added in the diets for all of the six groups. (Taurine amino) cinnamylaldehyde was added at 200, 300, 400, 500, and 600 g/ton for test groups 1, 2, 3, 4 and 5, respectively. No cinnamylaldehyde was added for the control group. The test was conducted from March 23, 2018, to April 4, 2018 in Chongqing city. The results are shown in Table 5.

**Table 5: Test on replacing the high amount of zinc and antibiotics with (taurine amino) cinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamylaldehyde in g/t | 200 | 300 | 400 | 500 | 600 | 0 |
| Zinc oxide in ppm | 1,500 | 1,500 | 0 | 0 | 0 | 2,250 |
| Quinoacetone in mg/kg | 50 | 50 | 50 | 50 | 50 | Quinoacetone 50 Nosiheptide 20 Kitasamycin 55 |
| Initial weight in kg | 6.33 | 6.41 | 6.38 | 6.36 | 6.34 | 6.35 |
| Final weight in kg | 10.35 | 10.62 | 10.32 | 10.62 | 10.49 | 10.16 |
| Daily weight gain (g) | 335 | 351 | 328 | 355 | 346 | 318 |
| Increase compared to the control group % | 5.35 | 10.38 | 3.14 | 11.64 | 8.80 | |
| Daily feed intake (g) | 363 | 378 | 350 | 370 | 386 | 348 |
| Feed/gain ratio | 1.08 | 1.08 | 1.07 | 1.04 | 1.12 | 1.09 |
| Incidence of diarrhea in % | 4.0 | 3.0 | 2.83 | 3.0 | 3.67 | 4.96 |

As can be seen from Table 5, when the 21 days old weaned piglets were fed with acidulant free diets with partially high amount of zinc (1500 ppm) at a suitable growth temperature (24°C), the diets with 200 mg/kg of cinnamylaldehyde and 300 mg/kg of cinnamylaldehyde were better than that with zinc at a high amount of 2,250 ppm, and the daily gain was increased by 5.35% and 10.38% respectively, in which 300 mg/kg of cinnamylaldehyde was preferred. When the 21 days old weaned piglets were fed with acidulant free diets without high amount of zinc (i.e., only 60 ppm of zinc), the diets with 400 mg/kg of cinnamylaldehyde, 500 mg/kg of cinnamylaldehyde and 600 mg/kg of cinnamylaldehyde were better than that with zinc at a high amount of 2,250 ppm, and the daily gain was increased by 3.14%, 11.64% and 8.80% respectively, in which 500 mg/kg of cinnamylaldehyde was preferred. Although the group with addition of 600 mg/kg of cinnamylaldehyde was better than the control group with addition of high amount of zinc, possibly due to the high concentration of cinnamaldehyde may affect the growth of piglet osteoblast, the growth effect and anti-diarrhea strength of the piglets were slightly worse than the group with addition of 500 mg/kg of cinnamylaldehyde. This example shows that stabilized cinnamylaldehyde in a feed can partially or compeletly replace the high amount of zinc.

### Example 5

240 21 days old weaned primiparous three-way crossbred piglets were divided into six groups, in which those having diarrhea (diseases) were not treated. The pig houses were in a natural temperature of 14-29°C. Except for zinc oxide, the basal diet, vitamin and trace elements for all of the six groups were given as shown in Table 1, 2 and 3, and acidulant was added in the diets for all of the six groups. Among them, test groups 1 and 2 were the partially high zinc groups using 1500 ppm of zinc (zinc oxide), with addition of 300 and 400 mg/kg of (lysine amino) cinnamylaldehyde respectively. Test groups 3, 4, and 5 did not use zinc oxide at all, with all addition of 500 mg/kg of (lysine amino) cinnamylaldehyde, and with addition of 200, 250, and 300 mg/kg of allantoin respectively. The piglets were fed for 12 days, and the test was conducted from March 27, 2018 to April 6, 2018, in Chongqing city. The results are shown in Table 6.

**Table 6: Test on replacing the high amount of zinc and antibiotics with lysine aminocinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamylaldehyde in g/t | 300 | 400 | 500 | 500 | 500 | 0 |
| Allantoin in g/t | | | 200 | 250 | 300 | |
| Zinc oxide ppm | 1,500 | 1,500 | 0 | 0 | 0 | 2,250 |
| Antibiotics mg/kg | | | | | | Virginiamycin 40 Aureomycin 75 Kitasamycin 55 |
| Initial weight in kg | 6.34 | 6.43 | 6.31 | 6.37 | 6.32 | 6.39 |
| Final weight in kg | 10.16 | 10.32 | 10.26 | 10.54 | 10.75 | 9.94. |
| Daily weight gain (g) | 318 | 324 | 329 | 348 | 369 | 296 |
| Increase compared to the control group % | 7.43 | 9.5 | 11.1 | 17.6 | 24.7 | |
| Daily feed intake (g) | 359 | 360 | 359 | 367 | 376 | 343 |
| Feed/gain ratio | 1.13 | 1.11 | 1.09 | 1.06 | 1.02 | 1.16 |
| Incidence of diarrhoea % | 5.3 | 4.4 | 4.7 | 3.9 | 3.1 | 6.9 |
| Dead and eliminated peiglets (piglets) | 1 | | | | | 2 |

As can be seen from Table 6, when the 21 days old weaned piglets were fed with acidulant-containing diets with partially high amount of zinc (1500 ppm) at a nature temperature of 15-29°C, the diets with 300 mg/kg of cinnamylaldehyde and 400 mg/kg of cinnamylaldehyde were better than that with zinc at a high amount of 2,250 ppm, and the daily gain was increased by 7.43% and 9.5% respectively, in which 400 mg/kg of cinnamylaldehyde was preferred. When the 21 days old weaned piglets were fed with acidulant-containing diets without high amount of zinc (only 60 ppm of zinc), the diets with 500 mg/kg of cinnamylaldehyde+200mg/kg of allantoin, 500 mg/kg of cinnamylaldehyde+250mg/kg of allantoin, and 500 mg/kg of cinnamylaldehyde+300mg/kg of allantoin in the test groups 3, 4 and 5 were all better than that with zinc at a high amount of 2,250 ppm, and the daily gain was increased by 11.1%, 17.6%, and 24.7% respectively, in which 500 mg/kg of cinnamylaldehyde+300mg/kg of allantoin was preferred. Although the group with addition of 600 mg/kg of cinnamylaldehyde in example 4 was better than the control group with addition of high amount of zinc, possibly due to the high concentration of cinnamaldehyde may affect the growth of piglet osteoblast, the growth effect and anti-diarrhea strength of the piglets were slightly worse than the group with addition of 500 mg/kg of cinnamylaldehyde. Thus, in this example, only 500 mg/kg of cinnamylaldehyde was added with addition of 200-300 mg/kg of allantoin, which achieved an effect far better than that of the high zinc control group, and the daily gain exceeded the high zinc control group by 11.1-24.7%. In practical applications, except that large-scale pig farms will be kept at a suitable temperature for pigs, most small and medium-sized pig farms will be kept at 15°C or above. This example has a strong practical guiding significance. Example 6 shows that a composition of stabilized cinnamylaldehyde and allantoin can partially or compeletly replace the high amount of zinc, in which the ratio of cinnamylaldehyde to allantoin can be any value, for example, cinnamylaldehyde : allantoin = 1-5000 : 1-5000, and preferably cinnamylaldehyde : allantoin = 1-1000: 1-1000.

### Example 6

240 21 days old weaned primiparous three-way crossbred piglets were divided into six groups, in which those having diarrhea (diseases) were not treated. The temperature of the pig houses were 22-25°C. Except for zinc oxide, the basal diet, vitamin and trace elements for all of the six groups were given as shown in Table 1, 2 and 3, and acidulant was added in the diets for all of the six groups. Glutamine aminocinnamylaldehyde was added for the test group, and no any antibiotics or high amount of zinc (60 ppm of zinc) was added for the test group 2, and antibiotics and zinc oxide were added for the other groups, as can be seen Table 8. The test was conducted from Aptil 18, 2018, to April 27, 2018 in Chongqing city. The results are shown in Table 7.

**Table 7 Test on replacing the high amount of zinc and antibiotics with glutamine aminocinnamylaldehyde**

| | Control group | Test 1 | Test 2 | Test 3 | Test 4 | Test 5 |
|---|---|---|---|---|---|---|
| Number of piglets and groups | 4×10 | 4×10 | 4×10 | 4×10 | 4×10 | 4×10 |
| Zinc oxide in kg | 3 | | 2 | 2 | 2 | 2 |
| Cinnamy laldehyde in g/ton | | 500 | 150 | 200 | 200 | 200 |
| Antibiotics in ppm | Kitasamycin 50 | | Kitasamycin 50 | Virginiamycin 25 | Oxytetracycline calcium 200 | Virginiamycin 5 |
| | Oxytetracycline calcium 300 | | Quinoacetone 50 | Quinoacetone 50 | Quinoacetone 50 | Quinoacetone 50 |
| | Aureomycin 75 | | | | Kitasamycin 5 | |
| Acidulant in kg | 4 | 4 | 4 | 4 | 4 | 4 |
| Initial weight in kg | 6.50 | 6.50 | 6.54 | 6.49 | 6.51 | 6.51 |
| Final weight in kg | 10.37 | 10.32 | 10.35 | 10.51 | 10.33 | 10.39 |
| Daily weight gain in g | 387 | 382 | 381 | 402 | 382 | 388 |
| Daily feed intake in g | 420 | 415 | 421 | 429 | 418 | 421 |
| Feed/gain ratio | 1.085 | 1.086 | 1.105 | 1.067 | 1.094 | 1.085 |
| Incidence of diarrhea in % | 0.05 | 0.04 | 0.04 | 0.05 | 0.06 | 0.05 |
| Catalase in mmol/ml | 14.4 | 14.5 | 14.0 | 14.37 | 14.3 | 14.0 |
| SOD in U/ml | 46.1 | 45.5 | 44.6 | 47.4 | 47.9 | 46.2 |
| Malondialdehyde in U/ml | 3.253 | 3.133 | 2.84 | 2.92 | 3.397 | 3.023 |
| Glutathione peroxidase in U/ml | 798.9 | 792.1 | 779.5 | 783.8 | 813.0 | 812.1 |
| Total antioxidant capability in U/ml | 3.693 | 4.13 | 3.823 | 3.753 | 2.967 | 2.907 |

As can be seen from Table 7, when the temperature was kept at 22-25°C, glutamine aminocinnamylaldehyde at a high dose of 500 ppm can completely replace the high amount of zinc, and glutamine aminocinnamylaldehyde at a low dose of 150-200 ppm can partially replace the high amount of zinc with a zinc content of 1500 ppm rather than 2,250 ppm in the feed.

### Example 7

240 21 days old weaned primiparous three-way crossbred piglets were divided into six groups, in which those having diarrhea (diseases) were commonly treated. The pig houses were forced to cool down with air conditioning and ice, and the temperature was controlled at 10-14°C. Except for zinc oxide, the basal diet, vitamin and trace elements for all of the six groups were given as shown in Table 1, 2 and 3, and acidulant was added in the diets for all of the six groups. Among them, test groups 1 and 2 were the partially high zinc groups using 1500 ppm of zinc (zinc oxide), with addition of 300 and 400 mg/kg of (ethylenediamine amino) cinnamylaldehyde respectively. Test groups 3, 4, and 5 did not use high amount of zinc at all (containing 60ppm zinc), with all addition of 500 mg/kg of (ethylenediamine amino) cinnamylaldehyde, and with addition of 300, 350, and 400 mg/kg of allantoin respectively. The piglets were fed for 12 days. The results are shown in Table 8.

**Table 8 Test on replacing the high amount of zinc and antibiotics with (ethylenediamine amino) cinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamylaldehyde in g/t | 300 | 400 | 500 | 500 | 500 | 0 |
| Allantoin in g/t | | | 300 | 350 | 400 | |
| Zinc oxide in ppm | 1,500 | 1,500 | 0 | 0 | 0 | 2,250 |
| Antibiotics in mg/kg | | | | | | Nosiheptide 40 Oxytetracycline calcium 200 Kitasamycin 55 |
| Initial weight in kg | 6.45 | 6.48 | 6.42 | 6.56 | 6.39 | 6.68 |
| Final weight in kg | 9.29 | 9.38 | 9.25 | 9.48 | 9.55 | 9.37 |
| Daily weight gain (g) | 237 | 242 | 236 | 243 | 263 | 224 |
| Increase compared to the control group % | 5.8 | 8.0 | 5.4 | 8.5 | 17.4 | |
| Daily feed intake (g) | 291 | 293 | 302 | 296 | 297 | 293 |
| Feed/gain ratio | 1.23 | 1.21 | 1.28 | 1.22 | 1.13 | 1.31 |
| Incidence of diarrhea in % | 7.3 | 6.9 | 7.2 | 6.5 | 4.7 | 9.4 |
| Dead and eliminated peiglets (piglets) | 2 | 1 | 2 | 1 | 0 | 4 |

As can be seen from Table 8, when the 21 days old weaned piglets were fed with acidulant-containing diets with partially high amount of zinc (1500 ppm) at a nature temperature of 10-14°C, the diets with 300 mg/kg of cinnamylaldehyde and 400 mg/kg of cinnamylaldehyde were better than that with zinc at a high amount of 2,250 ppm, and the daily gain was increased by 5.8% and 8.0% respectively, in which 400 mg/kg of cinnamylaldehyde was preferred. When the 21 days old weaned piglets were fed with acidulant-containing diets without high amount of zinc (60 ppm of zinc), the diets with 500 mg/kg of cinnamylaldehyde+300mg/kg of allantoin, 500 mg/kg of cinnamylaldehyde+350mg/kg of allantoin, and 500 mg/kg of cinnamylaldehyde+400mg/kg of allantoin in the test groups 3, 4 and 5 were all better than that with zinc at a high amount of 2,250 ppm, and the daily gain was increased by 5.4%, 8.5%, and 17.4% respectively, in which 500 mg/kg of cinnamylaldehyde+400mg/kg of allantoin was preferred. Although the group with addition of 600 mg/kg of cinnamylaldehyde in example 4 was better than the control group with addition of high amount of zinc, possibly due to the high concentration of cinnamaldehyde may affect the growth of piglet osteoblast, the growth effect and anti-diarrhea strength of the piglets were slightly worse than the group with addition of 500 mg/kg of cinnamylaldehyde. Thus, in this example, only 500 mg/kg of cinnamylaldehyde was added with addition of 300-400 mg/kg of allantoin, which achieved an effect far better than that of the high zinc control group, and the daily gain exceeded the high zinc control group by 5.4-17.4%. In practical applications, only a small number of individual pig farms will sometimes only have a temperature of 10°C or above when the temperature is low. This example has a strong practical guiding significance. This example shows that the stabilization treated cinnamaldehyde and allantoin can partially replace high amount of zinc or completely replace high amount of zinc at low temperature or sudden temperature drop.

### Example 8

240 28 days old weaned primiparous three-way crossbred piglets were divided into six groups, in which those having diarrhea (diseases) were commonly treated. The pig houses were forced to cool down with air conditioning and ice, and the temperature was controlled at 0-9°C. Except for zinc oxide, the basal diet, vitamin and trace elements for all of the six groups were given as shown in Table 1, 2 and 3, and acidulant was added in the diets for all of the six groups. Among them, test groups 1 and 2 were the partially high zinc groups using 1500 ppm of zinc (zinc oxide), with addition of 300 and 400 mg/kg of (antimicrobial amino) cinnamylaldehyde respectively. Test groups 3, 4, and 5 were completely free of zinc oxide, with all addition of 500 mg/kg of (glutamine amino) cinnamylaldehyde, and with addition of 300, 350, and 400 mg/kg of allantoin respectively. The piglets were fed for 12 days. The results are shown in Table 9.

**Table 9 Test on replacing the high amount of zinc and antibiotics with (antimicrobial peptides amino) cinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamylaldehyde in g/t | 300 | 400 | 500 | 500 | 500 | 0 |
| Allantoin in g/t | | | 400 | 500 | 600 | |
| Zinc oxide in ppm | 1,500 | 1,500 | 0 | 0 | 0 | 2,250 |
| Antibiotics in mg/kg | | | | | | Nosiheptide 60 Virginiamycin 80 Kitasamycin 120 |
| Initial weight in kg | 7.45 | 7.35 | 7.32 | 7.42 | 7.39 | 7.55 |
| Final weight in kg | 8.13 | 8.23 | 7.98 | 8.35 | 8.43 | 7.93 |
| Daily weight gain (g) | 57 | 73 | 55 | 78 | 87 | 32 |
| Increase compared to the control group % | 78 | 128 | 72 | 144 | 172 | |
| Daily feed intake (g) | 95 | 120 | 93 | 126 | 135 | 56 |
| Feed/gain ratio | 1.66 | 1.64 | 1.69 | 1.61 | 1.55 | 1.76 |
| Incidence of diarrhea in % | 10.3 | 9.1 | 10.6 | 9.7 | 7.9 | 12.3 |
| Dead and eliminated peiglets (piglets) | 2 | 1 | 2 | 1 | 0 | 4 |

As can be seen from Table 9, when the 28 days old weaned piglets were fed with acidulant-containing diets with partially high amount of zinc (1500 ppm) at a temperature of 0-9°C, the diets with 300 mg/kg of cinnamylaldehyde and 400 mg/kg of cinnamylaldehyde were better than that with zinc at a high amount of 2,250 ppm, and the daily gain was increased by 78% and 128% respectively, in which 400 mg/kg of cinnamylaldehyde was preferred. When the 21 days old weaned piglets were fed with acidulant-containing diets without high amount of zinc (60 ppm of zinc), the diets with 500 mg/kg of cinnamylaldehyde+400mg/kg of allantoin, 500 mg/kg of cinnamylaldehyde+500mg/kg of allantoin, and 500 mg/kg of cinnamylaldehyde+600mg/kg of allantoin in the test groups 3, 4 and 5 were all better than that with zinc at a high amount of 2,250 ppm, and the daily gain was increased by 72%, 144%, and 172% respectively, in which 500 mg/kg of cinnamylaldehyde+600mg/kg of allantoin was preferred. Although the group with addition of 600 mg/kg of cinnamylaldehyde in example 4 was better than the control group with addition of high amount of zinc, possibly due to the high concentration of cinnamaldehyde may affect the growth of piglet osteoblast, the growth effect and anti-diarrhea strength of the piglets were slightly worse than the group with addition of 500 mg/kg of cinnamylaldehyde. Thus, in this example, only 500 mg/kg of cinnamylaldehyde was added, with addition of 400-600 mg/kg of allantoin, which achieved an effect far better than that of the high zinc control group, and the daily gain exceeded the high zinc control group by 72-172%. Few of the peiglets died or were eliminated, and the incidence of diarrhea was low.

In practical applications, only a small number of pig farms will sometimes have a temperature of 10°C or above when the temperature suddenly drops. The temperature of the pig house will never be lower than 0°C regardless of accidents or other reasons. Thus, this example has strong confidence and guiding significance to get rid of the misunderstandings that "zinc must be used in a high amount" in this field, which is new epoch-making milestone in the feed industry. This example shows that a composition of stabilized cinnamylaldehyde and allantoin can partially or compeletly replace the high amount of zinc when temperature is low or drops suddenly.

Examples 1-8 were creep feed tests, which were conducted at an allowable use stage of high amount of zinc according to the feed regulations of China, i.e. two weeks after weaning. After that, it was the nursery feed stage. At the nursery feed stage, high amount of zinc is not allowed according to the national feed regulations. However, in actual use, in order to prevent diarrhea, most of pig farms and feed factories may add high amount of zinc which is higher than the nutritional requirements. Before July 1, 2018, most of the feed factories added 2.5 kg of zinc oxide, i.e., 1,875 ppm of zinc + 60-110 ppm of zinc required for nutrition. Although the pigs are in the nursery feed stage, the feed factories may indicate on the nursery feed label "used for piglets in two weeks after weaning" to avoid legal risks. This is the unspoken rule of the industry. Because the feed law does not stipulate the weaning age of the piglets, which allows the feed users to freely determine, so there are loopholes that can be exploited. As described in the example 1, on the ninth day after weaning, currently most pig farms have actually entered the nursery feed stage, and there is still a great risk of diarrhea without high amount of zinc. This is the only way for most of the feed factories and breeding farms.

In practical applications, most pig farms in China use nature temperature at the nursery stage, while only a small number of large-scale breeding enterprises can keep the pigs at a suitable temperature of 20-27°C. Therefore, in the nursery feed stage, pigs weighing 8-35 kg, particularly 8-15 kg in the early stage of nursery feed, are almost all fed with 1,875 ppm of high amount of zinc, i.e., 2.5 kg of zinc oxide per ton of feed. Of course, after the new regulations are officially implemented, a maximum of 1,600 ppm of zinc can only be used, i.e., 2 kg of zinc oxide (1500 ppm of zinc) plus 60-110 ppm of zinc required for nutrition.

The national regulations stipulate that use of high amount of zinc is only limited in two weeks after weaning of piglet. In the past, all got around the regulations. However, as the national law enforcement is getting stronger and stronger, many large companies are looking for new solutions. Although coated zinc oxide, nano-zinc oxide, tannic acid and other types of products can achieve the effect of high zinc in dialysis, but the feed utilization rate and the growth rate of pigs will drop by 10-50%, which seriously affects the development of animal husbandry. Stabilized cinnamaldehyde can continuously replace zinc oxide, and piglets have better weight gain and feed utilization efficiency.

The following examples are nursery feed tests, in which the basal diets, vitamine, and trace elements are given as Table 10, Table 2 and Table 3. Except for zinc oxide, other components and nutritional indicators of the control group are the same as the test groups.

**Table 10 Basal diet formula of nursery feed**

| Raw material | Ratio in kg |
|---|---|
| Corn | 685 |
| Fermented soybean meal | 40 |
| Dehulled soybean meal | 200 |
| Streamed fish meal | 20 |
| Soybean oil | 15 |
| Premix | 40 |
| Total | 1,000 |

### Example 9

240 34 days old three-way crossbred piglets were divided into six groups, in which those having diarrhea (diseases) were not treated. The pig houses were in a natural temperature of 14-29°C. The test was conducted in Chongqing City. Except for zinc oxide, the basal diet, vitamin and trace elements for all of the six groups were given as shown in Tables 10, 2 and 3, and 2kg of acidulant was added in the diets for all of the six groups. Among them, test groups 1 and 2 were the partially high zinc groups (containing 1200 ppm of zinc), with addition of 60 mg/kg and 100 mg/kg of coated (chitosan amino) cinnamylaldehyde respectively. Test groups 3, 4, and 5 did not use zinc oxide at all (containing 60 ppm of zinc), with addition of 200 mg/kg, 300 mg/kg, and 400 mg/kg of coated (chitosan amino) cinnamylaldehyde respectively. The piglets were fed for 30 days. The results are shown in Table 11.

**Table 11 Test on replacing the high amount of zinc and antibiotics with coated (chitosan amino) cinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamylaldehyde in g/t | 60 | 100 | 200 | 300 | 400 | 0 |
| Zinc oxide in ppm | 1,200 | 1,200 | 0 | 0 | 0 | 1,875 |
| Antibiotics in mg/kg | Virginiamycin 10 | Virginiamycin 10 | | | | Virginiamycin 30 Kitasamycin 55 |
| Initial weight in kg | 10.33 | 10.31 | 10.43 | 10.43 | 10.52 | 10.60 |
| Final weight in kg | 30.13 | 30.56 | 30.17 | 30.62 | 31.01 | 28.6 |
| Daily weight gain (g) | 660 | 675 | 658 | 673 | 683 | 600 |
| Increase compared to the control group % | 10.0 | 13.3 | 9.67 | 12.17 | 13.8 | |
| Daily feed intake (g) | 876 | 876 | 904 | 913 | 917 | 896 |
| Feed/gain ratio | 1.33 | 1.30 | 1.37 | 1.36 | 1.34 | 1.49 |
| Incidence of diarrhoea% | 0.8 | 0.6 | 1.0 | 0.3 | 0.2 | 1.3 |

As can be seen from Table 11, when the 34 days old piglets were fed with acidulant-containing diets with partially high amount of zinc (containing 1200 ppm of zinc) at a natural temperature of 14-29°C, the diets with 60 mg/kg of cinnamylaldehyde and 100 mg/kg of cinnamylaldehyde were better than that with zinc at a high amount of 1,875 ppm, and the daily gain was increased by 10.0% and 13.3% respectively, in which 100 mg/kg of cinnamylaldehyde was preferred. When the 34 days old piglets were fed with acidulant-containing diets without high amount of zinc (containing 60 ppm of zinc), the diets with 200 mg/kg of cinnamylaldehyde, 300 mg/kg of cinnamylaldehyde, and 400 mg/kg of cinnamylaldehyde in the test groups 3, 4 and 5 were all better than that with zinc at a high amount of 1,875 ppm, and the daily gain was increased by 9.67%, 12.17%, and 13.8% respectively, in which 400 mg/kg of cinnamylaldehyde was preferred. Although the diets with high amount of zinc can well control diarrhea during the nursery period, they also seriously affect the growth of pigs and are highly correlated with high zinc concentrations. This example shows that the stabilization treated cinnamaldehyde can partially or completely replace high amount of zinc.

### Example 10

240 34 days old three-way crossbred piglets were divided into six groups, in which those having diarrhea (diseases) were not treated. The pig houses were in a natural temperature of 14-29°C. The test was conducted in Chongqing City. Except for zinc oxide, the basal diet, vitamin and trace elements for all of the six groups were given as shown in Tables 10, 2 and 3, and 2kg of acidulant was added in the diets for all of the six groups. Among them, test groups 1 and 2 were the partially high zinc groups (containing 1200 ppm of zinc), with addition of 30 mg/kg and 60 mg/kg of allantoin respectively. Test groups 3, 4, and 5 did not use zinc oxide at all (containing 60 ppm of zinc), with addition of 100 mg/kg, 200 mg/kg, and 300 mg/kg of allantoin respectively. The piglets were fed for 30 days. The results are shown in Table 12.

**Table 12 Test on replacing the high amount of zinc with allantoin**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Allantoin in g/t | 30 | 60 | 100 | 200 | 300 | 0 |
| Zinc oxide in ppm | 1,200 | 1,200 | 0 | 0 | 0 | 1,875 |
| Antibiotics in mg/kg | Virginiamycin 10 | Virginiamyci n 10 | Virginiamyci n 20 | Virginiam ycin 20 | Virginiam ycin 20 | Virginiamycin 30 Kitasamycin 55 |
| Initial weight in kg | 10.32 | 10.34 | 10.33 | 10.40 | 10.42 | 10.50 |
| Final weight in kg | 29.05 | 29.24 | 29.33 | 29.85 | 29.50 | 28.2 |
| Daily weight gain (g) | 624 | 630 | 633 | 648 | 636 | 590 |
| Increase compared to the control group % | 5.8 | 6.8 | 7.3 | 9.8 | 7.8 | |
| Daily feed intake (g) | 911 | 907 | 899 | 927 | 922 | 920 |
| Feed/gain ratio | 1.46 | 1.44 | 1.42 | 1.43 | 1.45 | 1.56 |
| Incidence of diarrhoea% | 1.2 | 0.9 | 1.1 | 0.7 | 0.4 | 1.0 |

As can be seen from Table 12, when the 34 days old piglets were fed with acidulant-containing diets with partially high amount of zinc (containing 1200 ppm of zinc) at a natural temperature of 14-29°C, the diets with 30 mg/kg of allantoin and 60 mg/kg of allantoin were better than that with zinc at a high amount of 1,875 ppm, and the daily gain was increased by 5.8 % and 6.8 % respectively, in which 60 mg/kg of allantoin was preferred. When the 34 days old piglets were fed with acidulant-containing diets without high amount of zinc (containing 60 ppm of zinc), the diets with 100 mg/kg of allantoin, 200 mg/kg of allantoin, and 300 mg/kg of allantoin in the test groups 3, 4 and 5 were all better than that with zinc at a high amount of 1,875 ppm, and the daily gain was increased by 7.3%, 9.8%, and 7.8% respectively, in which 200 mg/kg of allantoin was preferred. This example shows that allantoin can partially or compeletly replace the high amount of zinc, but allantoin is less effective than cinnamylaldehyde to increase the weight of the pigs in nursery feed stage.

### Example 11

240 34 days old three-way crossbred piglets were divided into six groups, in which those having diarrhea (diseases) were not treated. The pig houses were controlled in a temperature of 20-25°C. The test was conducted in Chongqing City. Except for zinc oxide, the basal diet, vitamin and trace elements for all of the six groups were given as shown in Tables 10, 2 and 3, and 2kg of acidulant was added in the diets for all of the six groups. Among them, test groups 1 and 2 were the partially high zinc groups (containing 1200 ppm of zinc), with addition of 100 mg/kg and 150 mg/kg of cinnamylaldehyde commonly coated with polyacrylic acid resin respectively. Test groups 3, 4, and 5 did not use high amount of zinc at all (containing 60 ppm of zinc), with addition of 200 mg/kg, 300 mg/kg, and 400 mg/kg of commonly coated cinnamylaldehyde respectively. The piglets were fed for 30 days. The results are shown in Table 13.

**Table 13 Test on replacing the high amount of zinc and antibiotics with coated cinnamylaldehyde**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Cinnamy laldehyde in g/t | 100 | 150 | 200 | 300 | 400 | 0 |
| Zinc oxide in ppm | 1,200 | 1,200 | 0 | 0 | 0 | 1,875 |
| Antibiotics in mg/kg | Virginiamycin 10 | Virginiamycin 10 | | | | Virginiamycin 30 Kitasamycin 55 |
| Initial weight in kg | 10.30 | 10.33 | 10.41 | 10.34 | 10.40 | 10.51 |
| Final weight in kg | 29.71 | 30.10 | 30.99 | 28.30 | 30.68 | 29.30 |
| Daily weight gain (g) | 647 | 659 | 686 | 597 | 676 | 626 |
| Increase compared to the control group % | 3.35 | 5.27 | 1.60 | -4.6 | 7.99 | |
| Daily feed intake (g) | 872 | 881 | 908 | 871 | 907 | 951 |
| Feed/gain ratio | 1.35 | 1.37 | 1.32 | 1.46 | 1.34 | 1.52 |
| Incidence of diarrhoea% | 0.5 | 0.2 | 0.6 | 1.9 | 0.2 | 0.3 |

As can be seen from Table 13, when the 34 days old nursery pigs were fed with acidulant-containing diets with partially high amount of zinc (containing 1200 ppm of zinc) at a natural temperature of 20-25°C, the diets with 100 mg/kg of cinnamylaldehyde and 150 mg/kg of cinnamylaldehyde were better than that with zinc at a high amount of 1,875 ppm, and the daily gain was increased by 3.35 % and 5.27 % respectively, in which 60 mg/kg of cinnamylaldehyde was preferred. When the 34 days old piglets were fed with acidulant-containing diets without high amount of zinc (containing 60 ppm of zinc), the diets with 200 mg/kg of cinnamylaldehyde, 400 mg/kg of cinnamylaldehyde in the test groups 3, 4 and 5 respectively were all better than that with zinc at a high amount of 1,875 ppm, and the daily gain was increased by 9.58 % and 7.99 % respectively, in which 200 mg/kg of cinnamylaldehyde was preferred. The 300mg/kg of cinnamylaldehyde test group was 4.6% worse than the high zinc control group, and only 50 ppm of cinnamylaldehyde in the feed was detected. This indicates that the coated cinnamylaldehyde is easily damaged in the feed pelleting, and thus the coated cinnamylaldehyde is risky as a feed additive. Although the diets with high amount of zinc can well control diarrhea during the nursery period, they also seriously affect the growth of pigs and are highly correlated with high zinc concentrations. The coated and stabilization treated cinnamylaldehyde that has an intact coating and can withstand high temperature in pelleting and feed storage can partially or compeletly replace the high amount of zinc.

### Example 12

240 34 days old three-way crossbred piglets were divided into six groups, in which those having diarrhea (diseases) were not treated. The pig houses were controlled in a temperature of 20-25°C. The test was conducted in Chongqing City. Except for zinc oxide, the basal diet, vitamin and trace elements for all of the six groups were given as shown in Tables 10, 2 and 3, and 2kg of acidulant was added in the diets for all of the six groups. Among them, test groups 1 and 2 were the partially high zinc groups (containing 1200 ppm of zinc), with addition of 10 mg/kg and 20 mg/kg of allantoin respectively. Test groups 3, 4, and 5 did not use high amount of zinc at all (containing 60 ppm of zinc), with addition of 100 mg/kg, 200 mg/kg, and 300 mg/kg of allantoin respectively. The control group used 2.5 kg of zinc oxide. The piglets were fed for 30 days. The results are shown in Table 14.

**Table 14 Test on replacing the high amount of zinc with allantoin**

| | Test group 1 | Test group 2 | Test group 3 | Test group 4 | Test group 5 | Control group |
|---|---|---|---|---|---|---|
| Number of piglets | 4×10 | 4×10 | 4×10 | 4×10 | 4×10 | 4×10 |
| Allantoin in g/t | 10 | 20 | 50 | 100 | 150 | 0 |
| Zinc oxide in ppm | 1,200 | 1,200 | 0 | 0 | 0 | 1,875 |
| Antibiotics in mg/kg | Virginiamy cin 20 | Virginiam ycin 20 | Virginiamy cin 20 | Virginiamy cin 20 | Virginiamy cin 20 | Virginiamy cin 20 |
| Initial weight in kg | 10.31 | 10.36 | 10.32 | 10.34 | 10.30 | 10.40 |
| Final weight in kg | 30.03 | 30.61 | 30.35 | 30.87 | 30.00 | 28.90 |
| Daily weight gain (g) | 666 | 675 | 668 | 684 | 657 | 617 |
| Increase compared to the control group % | 7.9 | 9.4 | 8.3 | 10.9 | 6.5 | |
| Daily feed intake (g) | 872 | 878 | 888 | 882 | 893 | 900 |
| Feed/gain ratio | 1.31 | 1.30 | 1.33 | 1.29 | 1.36 | 1.46 |
| Incidence of diarrhea % | 0.4 | 0.5 | 0.6 | 0.2 | 0.1 | 0.4 |

As can be seen from Table 14, when the 34 days old piglets were fed with acidulant-containing diets with partially high amount of zinc (containing 1200 ppm of zinc) at a temperature of 20-25°C, the diets with 10 mg/kg of allantoin and 20 mg/kg of allantoin were better than that with zinc at a high amount of 1,875 ppm, and the daily gain was increased by 7.9% and 9.4% respectively, in which 20 mg/kg of allantoin was preferred. When the 34 days old piglets were fed with acidulant-containing diets without high amount of zinc (containing 60 ppm of zinc), the diets with 50 mg/kg of allantoin, 100 mg/kg of allantoin and 150 mg/kg of allantoin, in the test groups 3, 4 and 5 were all better than that with zinc at a high amount of 1,875 ppm, and the daily gain was increased by 8.3%, 10.9%, and 6.5% respectively, in which 100 mg/kg of allantoin was preferred. This example shows that allantoin can partially or compeletly replace the high amount of zinc, but allantoin is less effective than cinnamylaldehyde to increase the weight of the pigs in nursery feed stage.

### Example 13

240 40 days old three-way crossbred piglets were divided into four groups, in which those having diarrhea (diseases) were commonly treated. The pig houses were forced to cool down at a temperature of 5-12°C. Except for zinc oxide, the basal diet, vitamin and trace elements for all of the four groups were given as shown in Table 10, 2 and 3, and 2kg of acidulant was added in the diets for all of the four groups. Among them, test groups 1, 2 and 3 did not use high amount of zinc at all (containing 60ppm zinc), with all addition of 100 mg/kg, 200 mg/kg, and 300 mg/kg of allantoin respectively. Test groups 1, 2 and 3 used Cargill's product "Cinergy (containing 9% stabilized cinnamaldehyde)" based on cinnamaldehyde (see Table 15). The piglets were fed for 30 days. The results are shown in Table 15.

**Table 15 Test on replacing the high amount of zinc with cinnamylaldehyde plus allantoin**

| | Test group 1 | Test group 2 | Test group 3 | Control group |
|---|---|---|---|---|
| Number of piglets | 6×10 | 6×10 | 6×10 | 6×10 |
| Allantoin in g/t | 200 | 300 | 400 | 0 |
| cinnamylaldehyde in g/t | 135 | 158 | 180 | |
| Zinc oxide in ppm | 0 | 0 | 0 | 1,875 |
| Antibiotics in mg/kg | | | | Virginiamycin 40 Kitasamycin 55 Aureomycin 75 |
| Initial weight in kg | 11.42 | 11.32 | 11.39 | 11.43 |
| Final weight in kg | 27.95 | 28.32 | 25.33 | 26.97 |
| Daily weight gain (g) | 551 | 567 | 465 | 518 |
| Increase compared to the control group % | 6.4 | 9.5 | -10.2 | |
| Daily feed intake (g) | 898 | 902 | 785 | 865 |
| Feed/gain ratio | 1.63 | 1.59 | 1.69 | 1.67 |
| Incidence of diarrhea % | 3.4 | 3.6 | 1.6 | 2.9 |

As can be seen from Table 15, when the 40 days old piglets were fed with acidulant-containing diets without using high amount of zinc at all (containing 60ppm zinc) at a forced controled temperature of 5-12°C, the diets with 200 mg/kg of allantoin + 135mg/kg of stabilized cinnamaldehyde, 300 mg/kg of allantoin+158 mg/kg of stabilized cinnamaldehyde in the test groups 1, 2 and 3 were better than that with zinc at a high amount of 1,875 ppm, and the daily gain was increased by 6.4% and 9.5% respectively, in which 300 mg/kg of allantoin+158 mg/kg of stabilized cinnamaldehyde was preferred. Although the 400mg/kg of allantoin+180mg/kg of stabilized cinnamaldehyde group did not have more diarrhea rate than the control group, the daily weight gain decreased by 10.2% compared with the high-zinc control group. This may be due to the addition of 2000mg/kg of "Cinergy" in the diets, in which thymol, carvacrol and the like are contained and affect the feed intake. The food intake decreased by 9.25% compared with the control group. This example shows that the compostion of ("Cinergy") cinnamaldehyde, which is stabilized with solvent and β-cyclodextrin, and allantoin can also compeletly replace high amount of zinc and obtain slightly better effects than high amount of zinc, but cost of such replacement is much higher than the cost of high amount of zinc.

Based on the above description of the disclosed examples, those skilled in the art can implement or carry out the present disclosure. It is apparent for those skilled in the art to make many modifications to these examples. The general principle defined herein may be applied to other examples without departing from the spirit or scope of the present disclosure. Therefore, the present disclosure is not limited to the examples illustrated herein, but should conform to the widest scope consistent with the principle and novel features disclosed herein.

## Claims

1. Use of cinnamylaldehyde and/or allantoin for partially or completely replacing high amount of zinc in a piglet feed.

2. Use of stabilized cinnamylaldehyde and/or allantoin for partially or completely replacing high amount of zinc in a piglet feed.

3. A feed additive or additive premix for partially replacing high amount of zinc, based on the amount of complete formula feed, comprising 111-1600 ppm of zinc, and further comprising any one of a composition of 60-5000 ppm of cinnamylaldehyde and 10-5000 ppm of allantoin, 60-5000 ppm of cinnamylaldehyde, and 10-5000 ppm of allantoin.

4. The feed additive or additive premix according to claim 3, based on the amount of complete formula feed, comprising 111-1600 ppm of zinc, and further comprising any one of a composition of 60-600 ppm of cinnamylaldehyde and 10-400 ppm of allantoin, 60-600 ppm of cinnamylaldehyde, and 10-400 ppm of allantoin.

5. A feed additive or additive premix for completely replacing high amount of zinc, based on the amount of complete formula feed, comprising any one of a composition of 60-5000 ppm of cinnamylaldehyde and 10-5000 ppm of allantoin, 60-5000 ppm of cinnamylaldehyde, and 10-5000 ppm of allantoin.

6. The feed additive or additive premix according to claim 5, based on the amount of complete formula feed, comprising any one of a composition of 60-600 ppm of cinnamylaldehyde and 10-400 ppm of allantoin, 200-600 ppm of cinnamylaldehyde, and 50-400 ppm of allantoin.

7. The feed additive or additive premix according to any one of claims 3-6, wherein the cinnamylaldehyde is stablized cinnamylaldehyde; and
the stablized cinnamylaldehyde is specifically any one or more selected from coated cinnamylaldehyde, chemically modified cinnamylaldehyde, and chemically modified and coated cinnamylaldehyde.

8. The feed additive or additive premix according to claim 7, wherein
the coated cinnamylaldehyde is specifically obtained by coating cinnamylaldehyde with a coating material;
the chemically modified cinnamylaldehyde is specifically produced by condensing cinnamylaldehyde with a compound containing an amino or imino group; and
the chemically modified and coated cinnamylaldehyde is specifically obtained by condensing cinnamylaldehyde with a compound containing an amino or imino group and then coating with a coating material.

9. The feed additive or additive premix according to claim 8, wherein the compound containing an amino or imino group is specifically any one selected from oligochitosan, chitosan, lysine, methionine, threonine, tryptophan, glycine, aspartic acid, ethylenediamine, astragalus polysaccharide, cysteamine, glutamic acid, glutamine, antimicrobial peptides, aneurine hydrochloride, L-carnitine, DL-carnitine, niacinamide, ammonium bicarbonate, ammonium dihydrogen phosphate, diammonium hydrogen phosphate, isobutylidene diurea, urea phosphate, ammonium chloride, ammonium alginate, arginine, valine, leucine, isoleucine, methionine hydroxy analogue, tyrosine, taurine, N-carbamylglutamate, histidine, proline, phenylalanine, thiamine nitrate, cysteine, isobutylidene diurea, alginate oligosaccharide, nosiheptide, zinc bacitracin, amprolium hydrochloride ethoxy amide, robenidine hydrochloride, dinitolmide, bacitracin methylene disalicylate, xylooligosaccharide, chitosan oligosaccharide, galactomannan oligosaccharide, fructo-oligosaccharide, mannan oligosaccharide, alpha-cycloalanine, N,O-carboxymethyl oligochitosan, oligo β-(1,4)-2-amino-2-deoxy-glucose, guanidinoacetic acid, 8-methyl-N-vanillyl-L-nonenamide, capsaicine, dihydrocapsaicin, nordihydrocapsaicin, homocapsaicin, homodihydrocapsaicin I, homodihydrocapsaicin II, nonoyl vanillylamide, decoyl vanillylamide, and capryl vanillylamide.

10. The feed additive or additive premix according to claim 8, wherein the coating material is one or more selected from cyclodextrin, polyethylene glycol, polyvinyl alcohol, polyacrylic resins, hydroxypropyl methylcellulose phthalate, hydroxypropyl methylcellulose acetate, ethyl succinate butyrate, tributyrin, chitosan, pullulan, shellac, polyvinyl alcohol acetate phthalate, cellulose and derivatives thereof, cellulose acetate phthalate, cellulose acetate 1,2,4-benzenetricarboxylate, hydroxypropyl methylcellulose 1,2,4-benzenetricarboxylate, cellulose acetate succinate, hydroxypropyl cellulose acetate succinate, methacrylate, methacrylic acid, acrylate, butyl acrylate, acrylic resins, ethyl cellulose, glycerol triacetate, vinyl propionate, poly(vinyl propionate), poly(vinyl acetate), poly(vinyl butyrate), polymers of vinyl carboxylate polymer, vinyl acrylate, polymers of vinylpyrrolidone and vinyl carboxylate, polymers of vinyl acrylate, vinyl methacrylate and polymers thereof, and ethyl o-benzoate.

11. A piglet feed, containing the feed additive or additive premix accoridng to any one of claims 3-10.
